# EUROPEAN PATENT SPECIFICATION

(11) **EP 3 415 129 B1**
(45) Date of publication and mention of the grant of the patent: **12.05.2021**
(21) Application number: 16892527.9
(22) Date of filing: 01.03.2016
(51) Int. Cl.: A61F 13/15, A61F 13/49, A61F 13/494, A61F 13/496, A61F 13/551

(54) **PANTS-TYPE ABSORBENT ARTICLE**
HOSENÄHNLICHER SAUGFÄHIGER ARTIKEL
ARTICLE ABSORBANT DE TYPE CULOTTE

(43) Date of publication of application: 19.12.2018
(73) Proprietor: Unicharm Corporation, Ehime 799-0111 (JP)
(72) Inventor: BABA, Toshimitsu, Kanonji-shi Kagawa 769-1602 (JP)
(74) Representative: Dolleymores
(86) International application number: PCT/JP2016/056316
(87) International publication number: WO 2017/149683

(56) References cited:
- EP-A2- 1 205 171
- WO-A1-2004/031053
- JP-A- 2003 250 826
- JP-A- 2005 500 868
- JP-A- 2009 061 046
- JP-A- 2014 004 258
- JP-A- 2015 134 020
- JP-A- 2015 532 183
- US-A1- 2002 123 730
- US-A1- 2003 062 113
- US-A1- 2003 062 113
- US-A1- 2004 129 592
- US-A1- 2014 113 793

## Description

### Technical Field

The present invention relates to a pull-on absorbent article.

### Background Art

A pull-on disposable diaper is known that includes an absorbent main body to absorb excrement, and front and back waist sections that are arranged around the waist of the wearer when the diaper is being worn. When such a pull-on disposable diaper is being distributed in the marketplace, portions of the front and back waist sections that project out to the lateral direction outside of the absorbent main body (also referred to as side panels or side flaps) are preferably folded to achieve a compact shape before packaging. For example, Patent Document 1 discloses a pull-on disposable diaper in which side flaps projecting out from the two lateral direction sides are folded in from the outside toward the inside in the lateral direction.

### Citation List

### Patent Literature

Patent Literature 1: JP 2002-136545A - EP 1205171 A2 is a patent family member.

US 2002/0123730 A1 discloses packaged garments having pre-fastened, refastenable seams tucked in a position to preserve fastener performance.

US 2014/0113793 A1, US 2003/0062113 A1 and US 2004/0129592 A1 also disclose disposable diapers which are folded.

### Summary of Invention

### Technical Problem

An ordinary pull-on disposable diaper includes side flaps formed by joining together two lateral direction end portions of a front waist section and a back waist section. In the pull-on disposable diaper of Patent Document 1, at the position of the join portions between the front waist section and the back waist section, the join portions unfortunately overlap with the positions of fold lines of the side flaps resulting from the side flaps being folded in from the outside toward the inside in the lateral direction. Stable fold line formation is accordingly difficult. This means that the folded-in state of the side flaps is also difficult to maintain, and it is difficult to maintain a compactly folded state for the diaper overall. For example, when a user who has purchased a product (diapers) in a packaged state takes the diapers out from the package, the side flaps readily open out, and problems such as loss of compactness arise.

In consideration of the above issues, an object of the invention is to provide a pull-on diaper in which a compactly folded state of the side flaps is easily maintained.

### Solution to Problem

The present invention provides the pull-on absorbent article of independent claim 1. The dependent claims specify preferred but optional features.

In order to achieve the above object, a main invention is a pull-on absorbent article having a height direction, a lateral direction, and a front-back direction intersecting with each other. The pull-on absorbent article includes an absorbent main body configured to absorb excrement, a front exterior member arranged at one end side of the absorbent main body, and a back exterior member arranged at another end side of the absorbent main body. The front exterior member and the back exterior member are joined together at a pair of join portions. At least a portion of the front exterior member and the back exterior member is folded in from outside to inside in the lateral direction. The pair of join portions where the front exterior member and the back exterior member are joined together are respectively positioned further outside in the lateral direction than inside ends in the lateral direction of the folded-in front exterior member and the folded-in back exterior member. Side flaps are configured by the front exterior member and the back exterior member, the side flaps being folded in at positions different to positions of the pair of join portions.

Other features of the present invention will become apparent from the present specification and accompanying drawings.

### Advantageous Effects of Invention

According to the present invention, a pull-on diaper can be provided in which a compactly folded state of the side flaps is easily maintained.

### Brief Description of Drawings

Fig. 1 is a schematic perspective diagram of a diaper 1, as viewed from a front side.
Fig. 2 is a plan view of the diaper 1 in an unfolded and extended state.
Fig. 3 is a schematic cross-sectional view taken along line A-A of Fig. 1.
Fig. 4 is a schematic cross-sectional view taken along line B-B of Fig. 1.
Fig. 5 is a diagram illustrating a manufacturing flow for manufacturing the diaper 1.
Fig. 6 is a schematic diagram to explain each of the processes when manufacturing the diaper 1.
Fig. 7 is a cross-sectional view to explain a folded-in state of side flaps 70.
Fig. 8 is a schematic side view illustrating an example of a configuration of a side flap fold-in mechanism 100.
Fig. 9A and Fig. 9B are diagrams to explain an example of a configuration of a fold-in means 130.
Fig. 10 is a diagram illustrating an enlargement of region X in Fig. 9B.
Fig. 11A is a schematic plan view of the diaper 1 in a state in which the side flaps 70 have been folded in, as viewed from the front side.
Fig. 11B is a schematic plan view of the diaper 1 in a state in which the side flaps 70 have been folded in, as viewed from the back side.

### Description of Embodiments

At least the following items are apparent from the present specification and accompanying drawings.

A pull-on absorbent article having a height direction, a lateral direction, and a front-back direction intersecting with each other. The pull-on absorbent article includes an absorbent main body configured to absorb excrement, a front exterior member arranged at one end side of the absorbent main body, and a back exterior member arranged at another end side of the absorbent main body, with the front exterior member and the back exterior member joined together at a pair of join portions. At least a portion of the front exterior member and the back exterior member is folded in from outside to inside in the lateral direction. Each of the pair of join portions where the front exterior member and the back exterior member are joined together is respectively positioned further outside in the lateral direction than an inside end in the lateral direction of the folded-in front exterior member and the folded-in back exterior member. Side flaps are configured by the front exterior member and the back exterior member, the side flaps being folded in at positions different to positions of the pair of join portions.

According to such a pull-on absorbent article, due to side flaps configured by the front exterior member and the back exterior member being folded in at positions different to positions of the pair of join portions, bending deformation of the side flaps is not liable to be impeded by the join portions, and fold lines are readily formed at the fold-in positions. The side flaps are accordingly firmly folded in from the outside toward the inside in the lateral direction, and a compactly folded state of the pull-on absorbent article is easily maintained.

In this pull-on absorbent article, preferably at least one of the front exterior member and the back exterior member includes a portion in the height direction where many layers of material are stacked and a portion in the height direction where few layers of material are stacked.

According to such a pull-on absorbent article, a fold line is easily formed due to the low bending rigidity of the region where there are few layers of the stacked material in the side flaps, which are configured from the front exterior member and the back exterior member. The folded-in state of the side flaps is accordingly easily maintained, and a compactly folded state of the pull-on absorbent article is easily maintained.

In this pull-on absorbent article, preferably at least one of the front exterior member and the back exterior member includes a portion at a middle region between two end portions in the height direction where a number of stacked layers of the material is fewer than at the two height direction end portions.

According to such a pull-on absorbent article, due to the low bending rigidity in the height direction middle region of the side flaps, fold lines are easily maintained in the middle region when the side flaps are folded inward. Thus, even suppose it were to be difficult to form fold lines in the two height direction end portions, firm fold lines would still be formable in the middle region, and the folded-in state would be easier to maintain for the side flaps overall.

In this pull-on absorbent article, preferably the absorbent main body includes an absorbent core configured by stacking layers of a material having liquid absorption properties, and preferably two end portions in the lateral direction of the absorbent core include a portion that overlaps with the middle region of the front exterior member and the back exterior member.

According to such a pull-on absorbent article, due to the arrangement in which the absorbent core having high rigidity overlaps with the height direction middle region of the side flaps having low rigidity, the side flaps are easily bent at the two lateral direction end portions of the absorbent core. This promotes deformation of the side flaps and the folded-in state thereof is easily maintained.

In such a pull-on absorbent article, preferably a pair of leg-band members are arranged at two sides of the absorbent main body in the lateral direction, wherein end portions in the height direction of the leg-band members are positioned further inside in the height direction than end portions in the height direction of the absorbent main body.

According to such a pull-on absorbent article, a region in the height direction of the pull-on absorbent article where the front exterior member and the back exterior member overlap with the leg-band members is narrower than a region therein where the front exterior member and the back exterior member overlap with the absorbent main body. Due to the region of the side flaps where layers of the leg-band members are present and a higher rigidity arises having only a small surface area, the side flaps are easily bent, and the pull-on absorbent article is more easily folded compactly.

In this pull-on absorbent article, preferably the absorbent main body includes an absorbent core configured from stacked layers of a material having liquid absorption properties, and preferably at least one of the front exterior member and the back exterior member includes an elastic region that stretches and contracts along the lateral direction closer to a skin side than the absorbent core.

According to such a pull-on absorbent article, there is a great difference in rigidity at lateral direction end portions of the absorbent core with respect to the region where the side flaps overlap with the absorbent core having high rigidity, which is a region not easily contracted. Contraction force due to the elastic region accordingly readily acts further to the skin side than the absorbent core. The side flaps are accordingly easily bent toward the skin side so as to wrap around the absorbent core, and the pull-on absorbent article is more easily folded compactly.

In this pull-on absorbent article, preferably a sheet member capable of stretching and contracting in the lateral direction is provided in the elastic region.

According to such a pull-on absorbent article, due to employing a planar sheet member of a stretchable nonwoven fabric or the like as the elastic member, elasticity is generated across the entire plane. Stress is accordingly distributed, so that rebound force to bending of the side flaps does not readily occur. Due to the side flaps being easier to bend, the pull-on absorbent article is more easily folded compactly.

Such a pull-on absorbent article preferably also includes a portion where the front exterior member and the back exterior member folded in from outside to inside in the lateral direction at one side in the lateral direction, overlaps in the front-back direction with the front exterior member and the back exterior member folded in from the outside to the inside in the lateral direction at another side in the lateral direction.

According to such a pull-on absorbent article, due to there being a region where the side flaps overlap with each other in the front-back direction when the front exterior member and the back exterior member (the side flaps) have been folded in, frictional force is more readily generated at the overlap region. The side flaps overlapping with each other in the front-back direction are accordingly not liable to become misaligned. The folded-in state of the side flaps is accordingly more easily maintained, and the pull-on absorbent article is more easily folded compactly.

In such a pull-on absorbent article, preferably the pair of join portions are positioned outside in the lateral direction of the portion where the front exterior member and the back exterior member folded in from the outside to the inside in the lateral direction at one side of the lateral direction one side overlaps in the front-back direction with the front exterior member and the back exterior member folded in from the outside to the inside in the lateral direction at the other side of the lateral direction.

According to such a pull-on absorbent article, the thickness of the pull-on absorbent article can be suppressed from increasing due to an offset in position between the join portions and overlapped portions at portions where the left and right side flaps are folded in so as to overlap, which are thick portions due to the overlapping material. This enables the side flaps to be folded thinner, making compact packaging of the pull-on absorbent article easier.

In this pull-on absorbent article, preferably a surface area of a portion where the front exterior member and the back exterior member are folded in between a front side and back side of the absorbent main body in the front-back direction at a waist opening side in the height direction, is larger than a surface area of a portion where the front exterior member and the back exterior member are folded in between a front side and back side of the absorbent main body in the front-back direction at a crotch side in the height direction.

According to such a pull-on absorbent article, a folded-in amount of the side flaps at the waist opening side of the pull-on absorbent article is larger than a folded-in amount of the side flaps at the crotch side thereof. The side flaps are accordingly more easily overlapped with each other in the front-back direction at the waist opening side when the side flaps are being folded inward. The folded-in state is accordingly more easily maintained at the waist opening side, suppressing unintended opening of the waist opening of the pull-on absorbent article from opening.

In this pull-on absorbent article, preferably the absorbent main body is contracted in the lateral direction by the elastic region that stretches and contracts along the lateral direction, and preferably an amount of contraction in the lateral direction of the absorbent main body at the back side is different to an amount of contraction in the lateral direction of the absorbent main body at the front side.

According to such a pull-on absorbent article, the fold-in position of the side flaps in the lateral direction is accordingly different at the back side and the front side when the side flaps are folded in along lateral direction edge profiles of the absorbent main body. The external appearance profile of the pull-on absorbent article is accordingly different at the front side to at the back side, enabling the front and back of the pull-on absorbent article to be easily discerned.

### ===Embodiments===

### <Basic Configuration of Diaper 1>

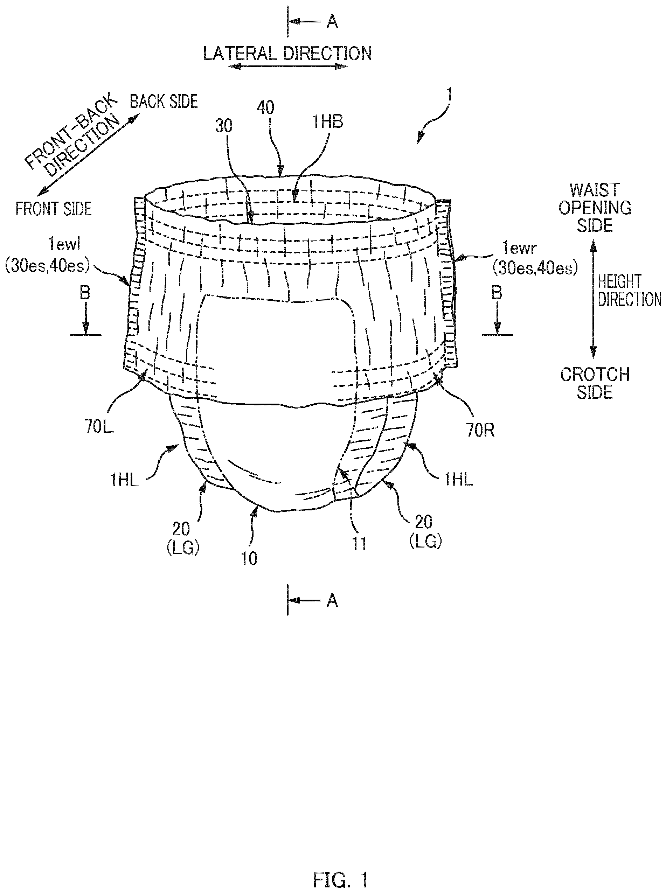

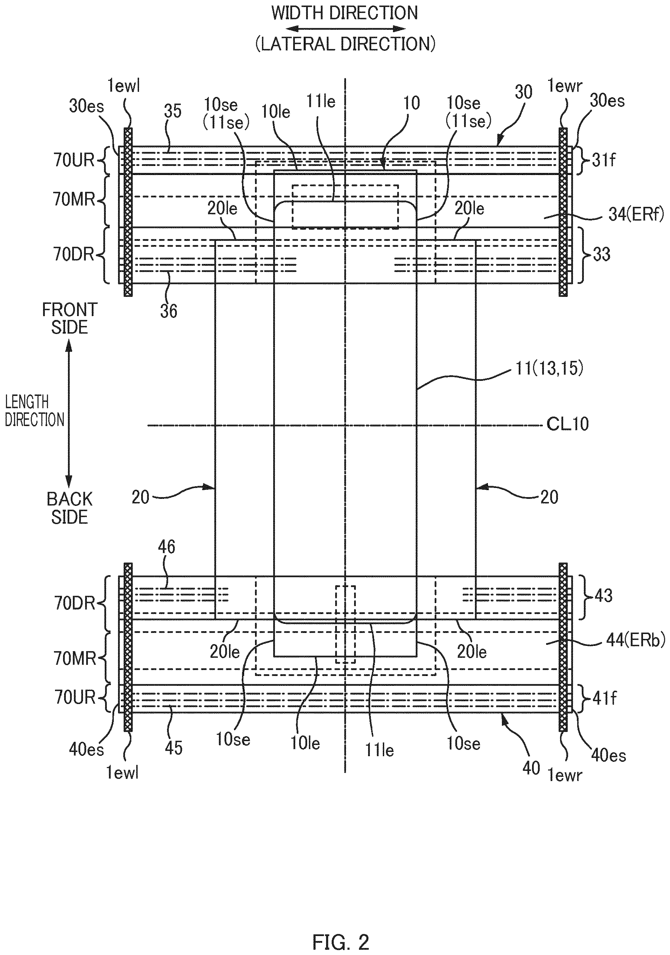

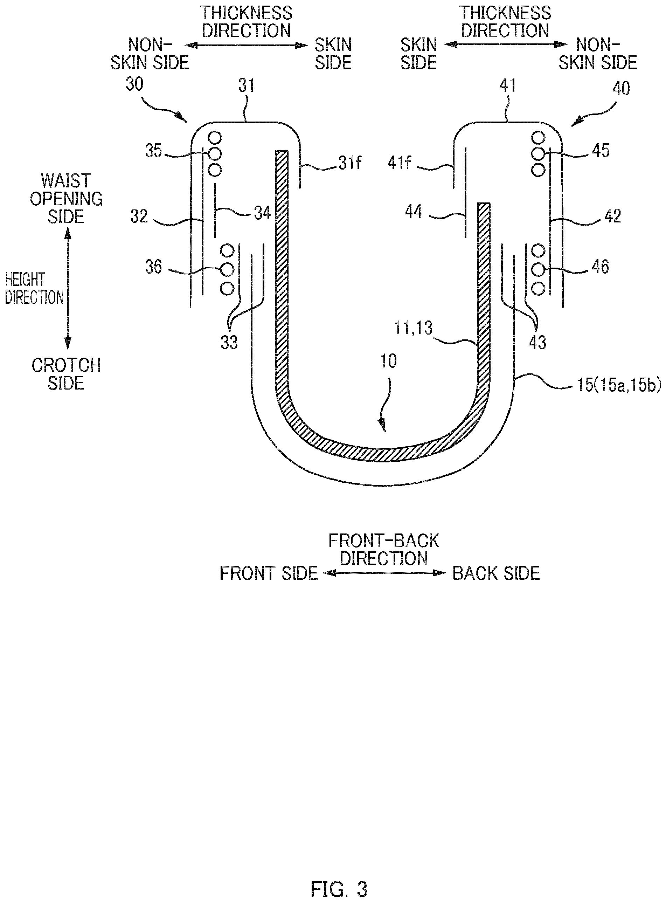

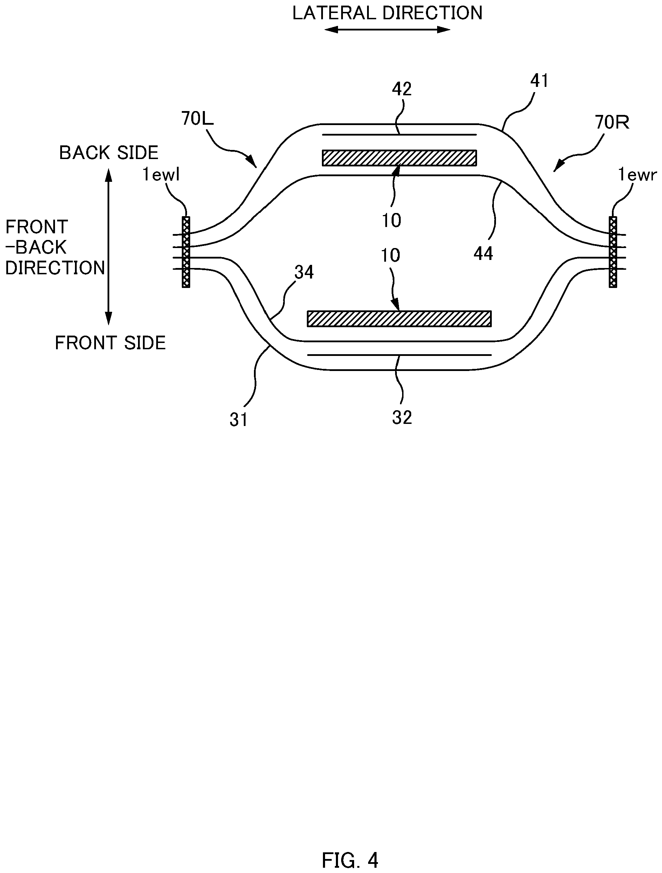

A description will first be given of a basic configuration of a pull-on disposable diaper 1 (also referred to below as "diaper 1"), serving as an example of an absorbent article handled by the present embodiment. Fig. 1 is a schematic perspective diagram of the diaper 1 as seen from a front side. Fig. 2 is a plan view of the diaper 1 in an unfolded and extended state. Fig. 3 is a schematic cross-section taken along line A-A of Fig. 1. Fig. 4 is a schematic cross-section taken along line B-B of Fig. 1. Note that the "unfolded and extended state" in Fig. 2 is a state in which a product (the diaper 1) has been extended so that there are no creases. Specifically, this could be described as a state extended such that the dimensions of each member configuring the diaper 1 have the same length as the dimensions of each of the individual parts themselves, or lengths that are close to such dimensions.

In the pants-style state of Fig. 1, the diaper 1 has three mutually orthogonal directions, a height direction, a lateral direction, and a front-back direction. In the following, one side and another side in the height direction of this pants-style state are referred to as the "waist opening side" and the "crotch side", and the front side and the back side in the front-back direction are referred to as the "front side" and the "back side".

In the unfolded state in Fig. 2, the diaper 1 has a length direction and a width direction of the three mutually orthogonal directions. In the following, one side and another side in the length direction of this unfolded state are also respectively referred to as the "front side" and the "back side". Note that the width direction in the unfolded state as referred to above is the same direction as the lateral direction referred to above with reference to the pants-style state. In the following the width direction is accordingly also sometimes referred to as the "lateral direction". Moreover, the length direction in the unfolded state is a direction along the height direction in the pants-style state. As illustrated in Fig. 3, a direction orthogonal to both the height direction (length direction) and the lateral direction (width direction) is referred to as the "thickness direction", and the side that contacts the skin of the wearer is referred to as the "skin side", and the opposite side thereto is referred to as the "non-skin side".

The diaper 1 of the present embodiment includes an absorbent main body 10, a leg-band member 20, a front exterior member 30, and a back exterior member 40. From the unfolded state of Fig. 2, the absorbent main body 10 is folded in two along a fold position at a predetermined position CL10 in the length direction (height direction) of the absorbent main body 10. The front exterior member 30 and the back exterior member 40 that face each other in this folded-in-two state are joined together by welding or the like at front side edges 30es and back side edges 40es, so as to form a right join portion 1ewr and a left join portion 1ewl. The front exterior member 30 and the back exterior member 40 are thereby joined together into a ring shape so as to form a pants-style state of the diaper 1 as illustrated in Fig. 1, with a waist opening 1HB and a pair of leg openings 1HL, 1LH.

The absorbent main body 10 has a function to absorb excrement such as urine. As illustrated in Fig. 2, the absorbent main body 10 has a substantially rectangular profile in plan view, and is arranged at the lateral direction center of the diaper 1, with the length direction of the absorbent main body 10 spanning along the height direction of the diaper 1. The absorbent main body 10 includes an absorbent core 11 with liquid absorption properties, a front-face sheet 13 that covers the absorbent core 11 from the skin side, and a back-face sheet 15 that covers the absorbent core 11 from the non-skin side and configures the exterior of the absorbent main body 10. The leg-band members 20 are also provided at each lateral direction side of the absorbent main body 10.

The absorbent core 11 is a member formed by layering a material with liquid absorption properties, and fibers with liquid absorption properties such as pulp fibers can, for example, be used therefor. Note that the absorbent core 11 may, for example, contain a highly absorbent polymer in liquid-absorbent granule form, and may include other materials with liquid absorption properties. The absorbent core 11 may be covered by a liquid permeable sheet such as tissue paper (not illustrated in the drawings).

The front-face sheet 13 is, for example, a liquid permeable nonwoven fabric having a plan view size larger than that of the absorbent core 11. The back-face sheet 15 is also a sheet having a plan view size larger than that of the absorbent core 11. An example thereof is a sheet having a double layer structure in which a leak prevention sheet 15a of a liquid impermeable material such as polyethylene or polypropylene is laminated to an exterior sheet 15b of a nonwoven fabric or the like. Moreover, what is referred to as a barrier cuff LSG (not illustrated in the drawings) may be formed to the absorbent main body 10 of the diaper 1. A barrier cuff LSG is a leak prevention wall erected at both lateral direction end portions of the absorbent main body 10.

The leg-band members 20 function as leg gathers LG, and are band-shaped sheet members arranged so as to run along the height direction at the two lateral direction end portions of the absorbent main body 10. The leg-band members 20 form a portion of the leg openings 1HL of the diaper 1. The leg-band members 20 are, for example, formed by folding a rectangular shaped nonwoven fabric along the height direction. Plural leg-band elastic members (not illustrated in the drawings) , such as rubber threads, are joined between the folded over nonwoven fabric while in a state extended along the height direction. Elasticity is imparted to the leg-band members 20 by the leg-band elastic members, improving the fitabilty of the leg openings 1HL. The length of the leg-band members 20 in the height direction (length direction) is shorter than the length of the absorbent main body 10 in the height direction (length direction) . Namely, the positions of end portions 201e at the two height direction ends of the leg-band members 20 are at the height direction inside of positions at end portions 101e at the two height direction sides of the absorbent main body 10.

The front exterior member 30 is joined to one end side of the absorbent main body 10 in the length direction (height direction) , and is a member that configures a waist section at the front side of the diaper 1. The front exterior member 30 of the present embodiment includes an exterior back sheet 31, a film sheet 32, cover sheets 33, a stretchable sheet 34, waist-gather elastic members 35, and fit-gather elastic members 36.

The exterior back sheet 31 is a sheet member made from a soft sheet material such as a nonwoven fabric and has a substantially rectangular shape in plan view. The height direction upper end portion of the exterior back sheet 31 is formed into a folded-over section 31f (see Fig. 3) by folding the exterior back sheet 31 from the height direction outside toward the height direction inside so as to be folded over in the thickness direction from the non-skin side toward the skin side (from the outside toward the inside in the front-back direction). The film sheet 32 is a sheet member made from a resin and printed with text, designs, and the like, and is arranged so as to be interposed in the thickness direction between the absorbent main body 10 and the exterior back sheet 31 (see Fig. 3 and Fig. 4) . The cover sheets 33 are band-shaped sheet members formed from a nonwoven fabric or the like, and are arranged along the lateral direction at a height direction lower end region of the exterior back sheet 31. The stretchable sheet 34 is a sheet member capable of exhibiting elasticity along the lateral direction, and is formed, for example, from a stretchable nonwoven fabric. The stretchable sheet 34 is arranged at a height direction central portion of the front exterior member 30 in a state extended along the lateral direction. The stretchable sheet 34 forms a front elastic region ERf that imparts elasticity in the lateral direction to the front exterior member 30.

The waist-gather elastic members 35 are elastic members such as rubber threads. Plural of the waist-gather elastic members 35 are joined to a height direction upper end portion of the front exterior member 30 in a state extended along the lateral direction and arranged between the exterior back sheet 31 and the folded-over section 31f. Elasticity is imparted to the waist opening 1HB of the diaper 1 by the elasticity of these waist-gather elastic members 35. The fit-gather elastic members 36 are elastic members such as rubber threads. Plural of the fit-gather elastic members 36 are joined to a height direction lower end portion of the front exterior member 30 in a state extended along the lateral direction and arranged between the exterior back sheet 31 and the cover sheets 33. Elasticity is imparted to a portion of the leg openings 1HL of the diaper 1 by the elasticity of the fit-gather elastic members 36. Note that, as illustrated in Fig. 2, some of the fit-gather elastic members 36 are cut or the like in a region where the fit-gather elastic members 36 overlap with the absorbent main body 10 in the thickness direction, so that this region does not exhibit elasticity. This suppresses the absorbent main body 10 from being excessively contracted in the lateral direction.

The back exterior member 40 is a member joined to the other length direction (height direction) end side of the absorbent main body 10, and configures a waistband section at the back side of the diaper 1. The back exterior member 40 of the present embodiment includes an exterior back sheet 41, a film sheet 42, cover sheets 43, a stretchable sheet 44, waist-gather elastic members 45, and fit-gather elastic members 46. The function and configuration of each member of the back exterior member 40 are substantially the same as those of the front exterior member 30 as described above (see Fig. 3 and Fig. 4), and so detailed description will be omitted thereof.

In the diaper 1, the proportional extension of the stretchable sheet 44 of the back exterior member 40 is set so as to be higher than the proportional extension of the stretchable sheet 34 of the front exterior member 30. Namely, in the diaper 1, the elasticity of a back elastic region ERb formed by the stretchable sheet 44 at the back side is greater than the elasticity of a front elastic region ERf formed by the stretchable sheet 34 at the front side. Thus results in the amount of contraction in the lateral direction of the absorbent main body 10 (the absorbent core 11) being different at the back side and the front side of the diaper 1. Specifically, the absorbent main body 10 is readily contracted in the lateral direction at the back side, and, as illustrated in Fig. 4, the width of the absorbent main body 10 in the lateral direction is wider on the front side. Furthermore, the size and shape of creases that develop on the front exterior member 30 and the back exterior member 40 on the back side are different from that on the front side.

Note that "proportional extension" indicates a degree of extension when the natural length of the elastic member (rubber thread/stretchable sheet) is taken as 1. For example, a proportional extension of 1.2 means that the elastic member is extended from its natural length by an amount of 0.2 times the natural length.

Moreover, in the following description, the portions of the front exterior member 30 and the back exterior member 40 of the diaper 1 that stick out to the outside in the lateral direction from the two lateral direction end portions of the absorbent main body 10 are referred to as a pair of side flaps 70. In Fig. 1, the diaper 1 includes a right side flap 70R sticking out to the right side, and a left side flap 70L sticking out to the left side.

### <Method for Manufacturing the Diaper 1>

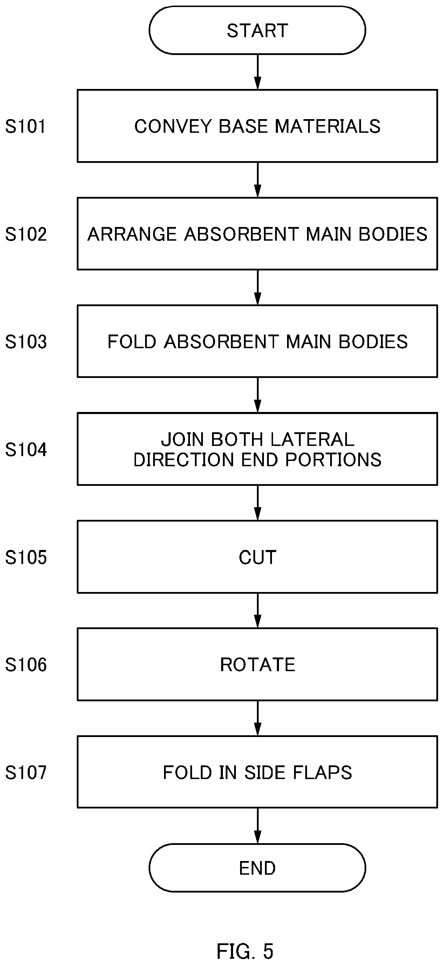

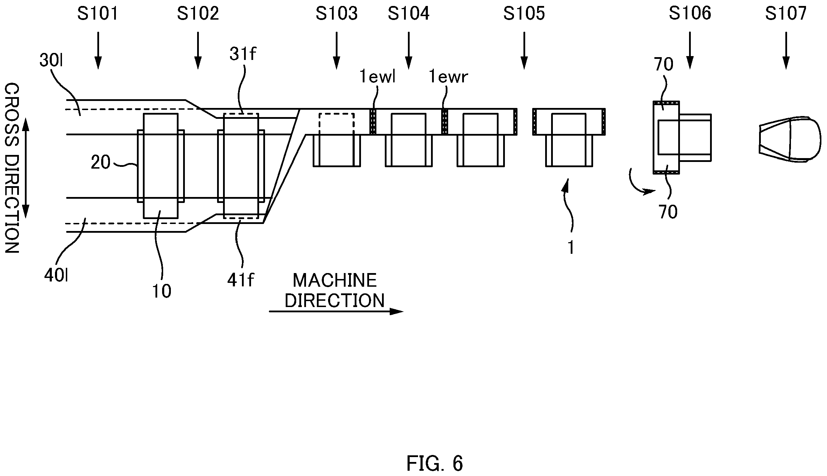

Description follows regarding an outline of a method for manufacturing the diaper 1. Fig. 5 is a diagram illustrating a production flow for manufacturing the diaper 1. Fig. 6 is a schematic diagram to explain each of the processes when manufacturing the diaper 1. The diaper 1 of the present embodiment is produced by successively executing each of the processes from S101 to S107 illustrated in Fig. 5 on a production line.

First, base materials for the diaper 1 are conveyed at a predetermined conveyance speed along a predetermined conveying direction (S101). Reference to "base materials of the diaper 1" means a front band-shaped member 301 configured by plural front exterior members 30 in a state connected together in the lateral direction, and a back band-shaped member 401 configured by plural back exterior members 40 in a state connected together in the lateral direction. These members are respectively conveyed in a conveying direction along the lateral direction (width direction in Fig. 2) while a positional relationship of a predetermined gap in the height direction (the length direction in Fig. 2) is held between these members. Such a conveying state is also called a "widthwise flow state". Moreover, in the following description a direction along the conveying direction will be called the Machine Direction, and a direction orthogonal to the Machine Direction will be called the Cross Direction.

Next, the absorbent main bodies 10 and the leg-band members 20 are arranged so as to span across in the Cross Direction between the front band-shaped member 301 and the back band-shaped member 401 as they are being conveyed in the Machine Direction, and the absorbent main bodies 10 and the leg-band members 20 are joined thereto (S102) . In the process of S102, a height direction end portion of the front band-shaped member 301 (the exterior back sheet 31) is folded over from the outside toward the inside, forming the folded-over section 31f. Similarly, a height direction end portion of the back band-shaped member 401 (the exterior back sheet 41) is folded over from the outside toward the inside, forming a folded-over section 41f. Each type of elastic member, such as the stretchable sheet 34 and the waist-gather elastic members 35, are appropriately arranged and joined by the processes of S102 and S101.

The absorbent main body 10 is then folded at a Cross Direction central portion (CL10) thereof, such that the front band-shaped member 301 and the back band back band-shaped member 401 are superimposed on each other in the thickness direction (S103) . The front band-shaped member 301 and the back band back band-shaped member 401 are then joined together at positions corresponding to the front side edges 30es (or the back side edges 40es) where the two lateral direction end portions of the front exterior member 30 (or of the back exterior member 40) will be formed. The right join portion 1ewr and the left join portion 1ewl are thereby formed (S104) . The front band-shaped member 301 and the back band back band-shaped member 401 are then cut at the positions of the two lateral direction end portions of the diaper 1 (namely, at positions of the front side edges 30es), thereby cutting and separating individual diapers 1 from the band-shaped member (S105).

The conveying direction of the cut and separated diapers 1 is then changed at a predetermined location on the conveying path. Specifically, the diapers 1 being conveyed along their lateral directions are rotated by 90 degrees with respect to the conveying direction (Machine Direction) as illustrated in Fig. 6, so as to be conveyed along their height directions (S106). This conveying state is also called a "lengthwise flow state". Namely, through the process of S106, the diapers 1 that were being conveyed in the widthwise flow state are to be conveyed in the lengthwise flow state. Note that although in the example of Fig. 6 the diapers 1 are turned such that the crotch sides thereof are pointing in the conveying direction, the diapers 1 may be turned such that the waist opening sides thereof are pointing in the conveying direction.

While the diapers 1 are being conveyed along their height directions, the side flaps 70L, 70R sticking out to the two lateral direction sides thereof are folded in from the outside toward the inside in the lateral direction (Cross Direction)(S107).

### Folding-in action performed on the side flaps 70

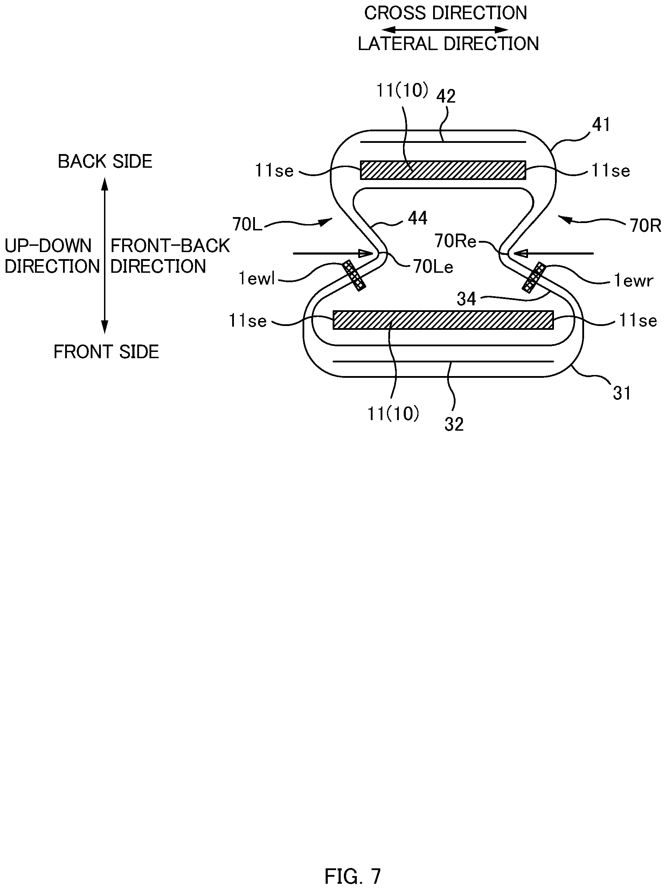

Fig. 7 is a cross-sectional view to explain a folded-in state of the side flaps 70. Fig. 7 illustrates a state in which the side flaps 70L, 70R are folded in inward in the lateral direction from the state illustrated in Fig. 4. In the folding-in process of S107 in Fig. 5, respective regions in the vicinity of the up-down direction (front-back direction) center of the side flaps 70L, 70R are pushed inward from the outside toward the inside in the lateral direction, so as to be folded in toward each other between the front side and the back side of absorbent main body 10 in the up-down direction, while the side flaps 70L, 70R are being deformed into substantially ∑ shapes, as illustrated in Fig. 7. The lateral direction size of the diapers 1 is thereby shortened, enabling compact folding and packaging of the diapers (product) 1.

In the diaper 1 of the first embodiment, the folded-in state of the side flaps 70 is easily maintained due to folding in the side flaps 70 from the outside toward the inside in the lateral direction at positions different from the positions of the pair of join portions 1ewl, 1ewr where the front exterior member 30 and the back exterior member 40 are joined together. Specifically, the left side flap 70L is folded inside in the lateral direction at the fold-in position 70Le different to the position of the left join portion 1ewl. As a result, as illustrated in Fig. 7, the fold-in position 70Le is positioned at a lateral direction inside end of the left side flap 70L when in the folded-in state, and the left join portion 1ewl is positioned at the lateral direction outside of the fold-in position 70Le. Similarly, the right side flap 70R is folded inside in the lateral direction at the fold-in position 70Re positioned at a different position to the right join portion 1ewr. The fold-in position 70Re is positioned at the lateral direction inside end of the right side flap 70R in the folded-in state, and the right join portion 1ewr is positioned to the lateral direction outside of the fold-in position 70Re.

The region where the join portions 1ewl, 1ewr are formed has a predetermined thickness due to welding or the like, and the rigidity thereof is greater than that of other regions of the side flaps 70. Thus in cases in which the positions of the join portions 1ewl, 1ewr overlap with the positions of the fold-in positions 70Le, 70Re, the side flaps 70L, 70R do not readily bend, and this tends to impede ∑ shaped deformation as illustrated in Fig. 7. Moreover, due to it being difficult to form fold lines at the fold-in positions 70Le, 70Re, even suppose that the side flaps 70L, 70R could be deformed into the ∑ shapes, they would soon return to their original shapes, with a concern that the folded-in state of the side flaps 70 would be difficult to maintain. In contrast thereto, such a problem does not tend to arise with the diapers 1 of the present embodiment, due to the positions of the join portions 1ewl, 1ewr and the positions of the fold-in positions 70Le, 70Re being offset from each other. This enables the side flaps 70 to be folded in firmly, and for the diapers 1 to be folded compactly. Moreover, a compact folded-in state of the side flaps 70 is more easily maintained.

The side flaps 70 (the front exterior member 30 and the back exterior member 40) of the diaper 1 also include portions in the height direction where there are many stacked layers of material and portions in the height direction where there are few stacked layers of material. For example, as illustrated in Fig. 2 and Fig. 3, the folded-over sections 31f, 41f are provided in a height direction upper (waist opening side) region of the front exterior member 30 and the back exterior member 40, and the waist-gather elastic members 35, 45 are also arranged therein. The region where the folded-over sections 31f, 41f are provided is referred to as upper region 70UR. The cover sheets 33, 44 are provided in a height direction lower (crotch side) region of the front exterior member 30 and the back exterior member 40, and the fit-gather elastic members 36, 46 are also arranged in this region. The region where the cover sheets 33, 44 are provided is referred to as the lower region 70DR. In contrast thereto, neither the folded-over sections 31f, 41f nor the cover sheets 33, 44 or the like are provided in a region (middle region 70MR) between the upper region 70UR and the lower region 70DR. There are accordingly fewer stacked layers of material configuring the side flaps 70 at the middle region 70MRthan at either the upper region 70UR or the lower region 70DR.

Due to bending rigidity being lower in regions where there are fewer stacked layers of material, fold lines are more easily formed in these regions when folding the side flaps 70 inwards, and so the folded-in state of the side flaps 70 that have been deformed into ∑ shapes is readily maintained. In particular, due to the lower bending rigidity in the side flaps 70 of the diapers 1 in the height direction middle region 70MR, the fold lines are more easily maintained in the middle region 70MR in the folded-in state of the side flaps 70. Thus, even suppose that fold lines were difficult to form in the upper region 70UR and the lower region 70DR, fold lines would be firmly formable in the middle region 70MR. The bent portions would accordingly not be liable to return to their original shape, making it easier to maintain the folded-in state of the side flaps 70 overall.

Note that when folding the side flaps 70 in as illustrated in Fig. 7, the side flaps 70 easily bend along the lateral direction end portions 11se of the absorbent core 11. The rigidity of the absorbent core 11 is relatively high compared with the side flaps 70 due to the stacked layers of fibers having liquid absorption properties, and so there is a great difference in rigidity between regions of the side flaps 70 overlapping with the absorbent core 11 and regions not overlapping the absorbent core 11. Namely, in the regions where the side flaps 70 and the absorbent core 11 overlap in the thickness direction, there is a great difference in rigidity at the boundary to the absorbent core 11 (at the peripheral edge of the absorbent core 11) . Thus, when the side flaps 70 have been folded inside in the lateral direction, the lateral direction end portions 11se of the absorbent core 11 where there is a great difference in rigidity tend to be the points where bending occurs.

Thus, in the present embodiment, at least a portion of the height direction end portions 11le of the absorbent core 11 is arranged in the middle region 70MR where the rigidity of the side flaps 70 is low. Namely, the absorbent core 11 includes a portion that overlaps with the middle region 70MR (see Fig. 2) . The side flaps 70 are more easily bent at these portions due to the difference in rigidity being particularly great in these portions overlapping the lateral direction end portions 11se of the absorbent core 11 (namely, at the boundary between the side flaps 70 and the absorbent core 11). Such a structure promotes ∑ shaped deformation of the side flaps 70, and also facilitates the maintenance of a folded-in state.

Moreover, although the leg-band members 20 are provided at both lateral direction sides of the absorbent main body 10 in the diapers 1, the height direction end portions 201e of the leg-band members 20 are positioned further inside in the height direction (length direction of the absorbent main body 10) than the height direction end portions 101e of the absorbent main body 10. Thus in the height direction of the diapers 1, the region where the front exterior member 30 and the back exterior member 40 overlap with the leg-band members 20 is narrower than the region where the front exterior member 30 and the back exterior member 40 overlap with the absorbent main body 10. Namely, the surface area of the region where the leg-band members 20 are stacked on the side flaps 70 is small, and a low rigidity region of the side flaps 70 is commensurately wider. This facilitates deformation of the side flaps 70 into ∑ shapes. Moreover, due to the leg-band members 20 not overlapping with the middle region 70MR as described above (see Fig. 2), a low rigidity is maintained for the middle region 70MR. This means that the leg-band members 20 are not liable to impede bending (∑ shaped deformation) of the side flaps 70, and the folded-in state of the side flaps 70 is easily maintained due to fold lines being formed in the middle region 70MR.

Moreover, in the diapers 1, the elastic region that stretches and contracts in the lateral direction is provided on the side flaps 70 at the skin side (front-back direction inside) of the exterior members. Specifically, a front elastic region ERf is formed by the stretchable sheet 34 on the skin side of the exterior back sheet 31 of the front exterior member 30, and a back elastic region ERb is formed by the stretchable sheet 44 at the skin side of the exterior back sheet 41 of the back exterior member 40. Namely, the side flaps 70 have a higher elasticity in the lateral direction in members of the side flaps 70 on the skin side in the thickness direction than members on the non-skin side thereof. When the skin side members (the stretchable sheets 34, 44) contract in the lateral direction, bending tends to occur toward the skin side such that the non-skin side members (the exterior back sheets 31, 41) tend to be pulled by the skin side members. The side flaps 70 are accordingly easily bent toward the skin side at the portions of the lateral direction end portions 11se of the absorbent core 11.

In particular, at the back side of the diapers 1, the back elastic region ERb (the stretchable sheet 44) is provided further to the skin side than the absorbent core 11. Contraction of the side flaps 70 (the back exterior member 40) is difficult in the region overlapping with the high rigidity absorbent core 11. The contraction force due to the back elastic region ERb readily acts further to the skin side than the absorbent core 11 due to the great difference in rigidity between the absorbent core 11 and the side flaps 70 at the lateral direction end portions 11se of the absorbent core 11. The side flaps 70 accordingly tend to bend toward the skin side at the lateral direction end portions 11se of the absorbent core 11 so as to wrap around the absorbent core 11. Note that the back elastic region ERf (the stretchable sheet 34) may be provided further to the skin side than the absorbent core 11 at the front side of the diapers 1.

In the diapers 1, the side flaps 70 bend easily due to the elastic regions ERf, ERb being formed by the stretchable sheets 34, 44 that are planar sheet members. In ordinary pull-on disposable diapers, often rubber threads are employed as elastic members to form the elastic regions. In cases in which rubber threads are used as elastic members, elasticity is generated along the portions where the rubber threads are arranged, and localized stress is accordingly high at these portions. This means that in cases in which rubber threads are provided in the side flaps 70, stress of the rubber thread acts as a rebound force when the side flaps 70 are bent, which sometimes makes it difficult to bend the side flaps 70. In contrast thereto, in cases in which a stretchable nonwoven fabric is employed as an elastic member as in the diapers 1, elasticity is generated across the entire plane, and stress is distributed. This means that localized rebound force as a reaction to bending of the side flaps 70 does not readily occur, and the side flaps 70 are more easily deformed into ∑ shapes.

### <Side Flap Folding-In Action>

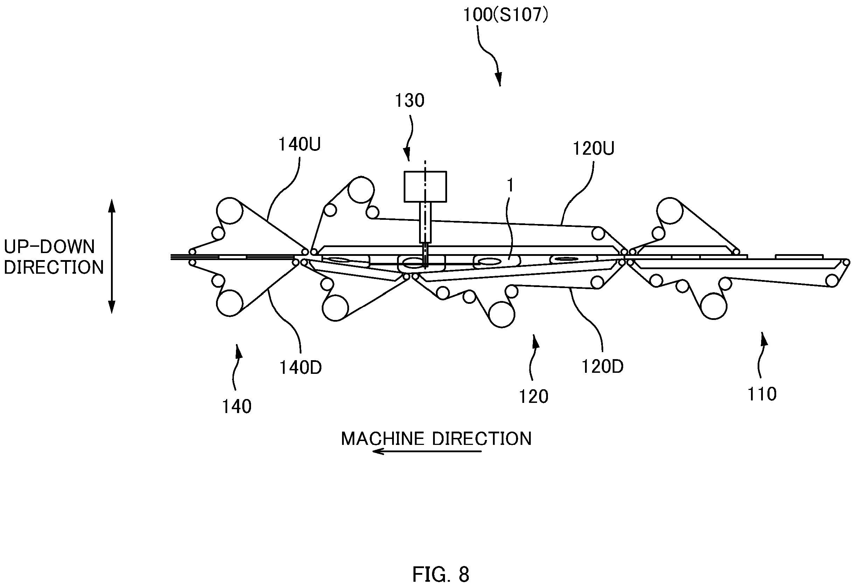

A specific action will now be described in a folding-in process of the side flaps 70 described with reference to S107 of Fig. 5. Fig. 8 is a schematic side view illustrating an example of a configuration of a side flap fold-in mechanism 100.

The side flap fold-in mechanism 100 includes a conveying means 110, a pulling means 120, a fold-in means 130, and a compressing means 140. The conveying means 110 has a function to convey the diapers 1 along the conveying direction (MD) and, for example, a suction conveyor is employed therefor. The suction conveyor includes a conveyor belt and a suction mechanism, and conveys the diapers 1 along the conveying direction (MD) in a lengthwise flow state while the diapers 1 are suctioned onto the surface of a belt conveyor and held thereon.

The pulling means 120 includes a function to open the waist opening 1HB (the side flaps 70) out in the front-back direction by pulling a front region and a back region of the absorbent main bodies 10 toward opposite sides in the up-down direction (the front-back direction of the diapers 1) as the diapers 1 are being conveyed along the Machine Direction. For the pulling means 120, a suction conveyor similar to the conveying means 110 may, for example, be employed. In the example of the pulling means 120 in Fig. 8, an upward suction conveyor 120U and a downward suction conveyor 120D are provided so as to be arranged facing toward each other in the up-down direction. The diapers 1 are then conveyed in a state in which the upward suction conveyor 120U suctions and holds a portion of the back region of each of the absorbent main bodies 10 upward, and the diapers 1 are conveyed in a state in which the downward suction conveyor 120D suctions and holds a portion at the front region of each of the absorbent main bodies 10 downward. Note that upward suction conveyor 120U and the downward suction conveyor 120D are configured to enable the diapers 1 to be respectively conveyed at the same speed along the Machine Direction.

In the present embodiment, the gap in the up-down direction between the upward suction conveyor 120U and the downward suction conveyor 120D widens on progression downstream in the conveying direction (Machine Direction). Specifically, as illustrated in Fig. 8, the conveying face of the downward suction conveyor 120D is arranged so as to be inclined with respect to the conveying face of the upward suction conveyor 120U. Thus the front region and the back region of the absorbent main body 10 are each pulled toward opposite sides in the up-down direction while the diapers 1 are being conveyed along the Machine Direction, thereby opening the waist opening 1HB formed by the side flaps 70 upward and downward (toward the front and back of the diapers 1). Note that the front-back direction of the diapers 1 as they are being conveyed may be the opposite to the situation described above. Namely, the front region of the absorbent main body 10 may be pulled upward, and the back region of the absorbent main body 10 may be pulled downward.

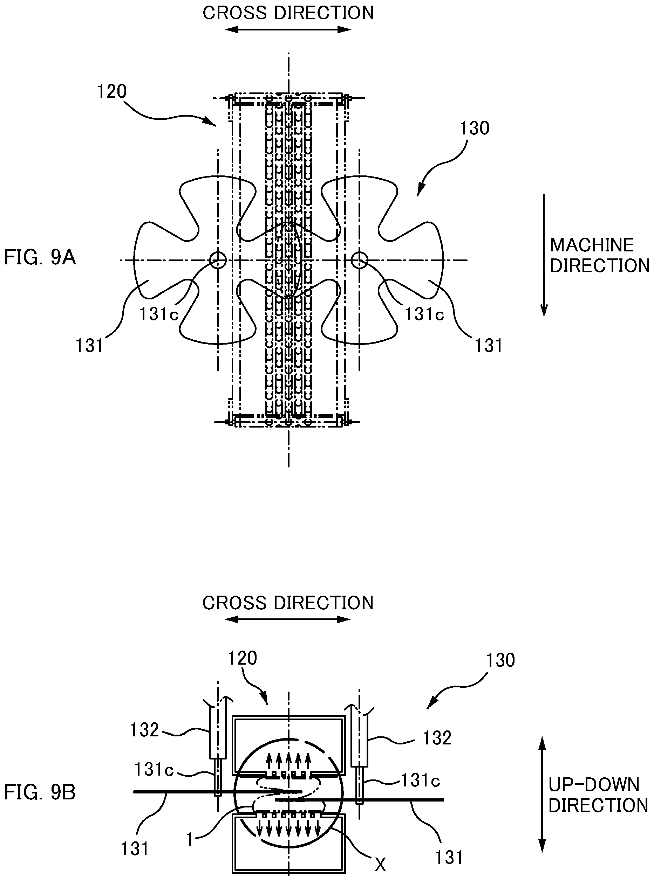

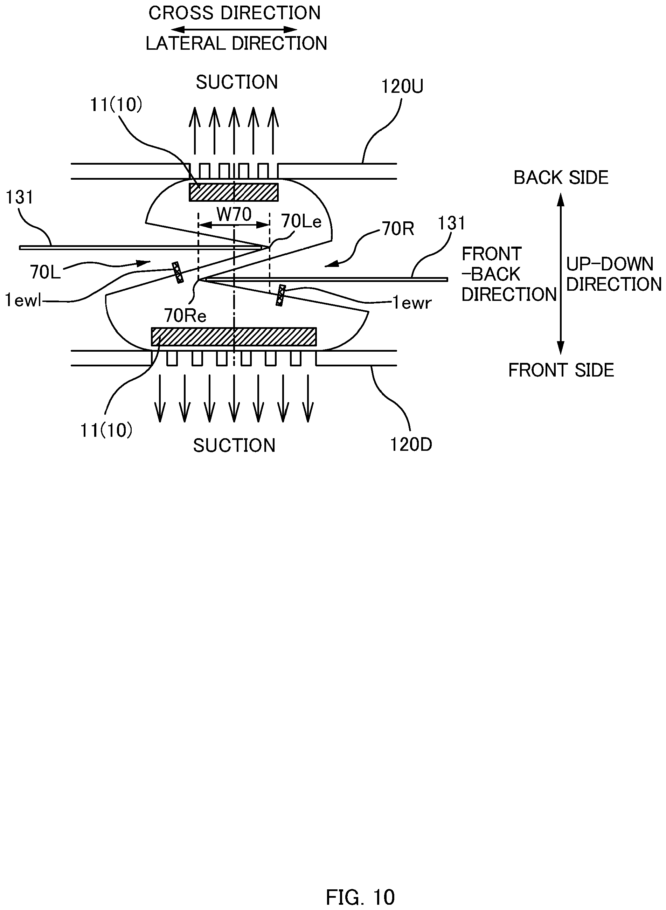

The fold-in means 130 has a function to respectively fold the side flaps 70L, 70R of the diapers 1 that have been opened out in the front-back direction (up-down direction) from the outside toward the inside in the lateral direction (Cross Direction) and, for example, an impeller may be employed therefor. Fig. 9A and Fig. 9B are diagrams to explain an example of a configuration of the fold-in means 130. Fig. 10 is a diagram illustrating an enlargement of region X in Fig. 9B. The fold-in means 130 of the present embodiment includes impellers 131 and impeller drive sections 132. The impellers 131 are configured by a pair of impellers provided on both Cross Direction sides of the pulling means 120, and each of the impellers rotates about a rotation center of a rotation shaft 131c disposed along the up-down direction. The impeller drive sections 132 are drive sections to rotationally drive the impellers 131.

As illustrated in Fig. 8, the fold-in means 130 is provided downstream of the pulling means 120 in the conveying direction. The rotationally driven impellers 131 push against the side flaps 70L, 70R of the diapers 1, now in a sufficiently opened state in the front-back direction (up-down direction) due to the pulling means 120, by pushing inward in the lateral direction (Cross Direction) from the outside toward the inside. The left and right side flaps 70L, 70R are thereby respectively deformed into ∑ shapes as illustrated in Fig. 9B, and the absorbent main body 10 is folded in between the front side and the back side thereof (namely, somewhere along the front-back direction).

In the fold-in means 130 of the present embodiment, the left and right impellers 131 provided at each Cross Direction side are arranged so their heights in the up-down direction are at different positions to each other. As illustrated in Fig. 10, in a state in which the side flaps 70L, 70R have been folded in the lateral direction (Cross Direction) to the furthest extent toward the inside, the right impeller 131 and the left impeller 131 are arranged such that portions of the respective impellers 131 partially overlap in the up-down direction. Such an arrangement enables the side flaps 70L, 70R to be folded inward more deeply. Specifically, in at least a part of a region of the left side flap 70L that has been folded inward to the maximum extent in the lateral direction (Cross Direction), the left side flap 70L is folded inward deeply to the extent that a fold-in position 70Le is positioned on the right side of a central position in the Cross Direction. Similarly, in at least a part of a region of the right side flap 70R that has been folded inward to the maximum extent in the lateral direction (Cross Direction), the right side flap 70R is folded inward deeply to the extent that a fold-in position 70Re is positioned on the left side of the central position in the Cross Direction. As a result of this, an overlap region W70 is formed where portions of the folded in left side flap 70L and right side flap 70R overlap with each other in the front-back direction (up-down direction). Frictional force arises in the overlap region W70 between the left side flap 70L and right side flap 70R that are overlapped in the front-back direction (up-down direction). These overlapped portions are accordingly not readily separated. Thus, even when the diapers 1 are conveyed further downstream than the position of the fold-in means 130, and the pushing in action by the impellers 131 toward the lateral direction inside no longer acts, the folded-in state of the side flaps 70 is easily maintained.

Note that the height in the up-down direction of the impellers 131 at the two Cross Direction sides is adjusted so as to enable a folding-in action to be performed smoothly on the side flaps 70L, 70R, and so as to facilitate the generation of friction when the side flaps 70L, 70R are in the folded-in state by causing the side flaps 70L, 70R to contact each other in the up-down direction in the overlap region W70.

The impellers 131 are then adjusted in the up-down direction so as to perform inward directed folding at different positions to the positions of the left join portion 1ewl and the right join portion 1ewr provided to the side flaps 70. Thus the left join portion 1ewl of the left side flap 70L is positioned further to the lateral direction outside than the fold-in position 70Le, and the right join portion 1ewr of the right side flap 70R is positioned further to the lateral direction outside than the fold-in position 70Re. Accordingly, as stated above, the fold lines are firmly formed at the fold-in positions 70Le, 70Re, and the folded-in state of the side flaps 70 is more easily maintained.

Moreover, the diapers 1 include portions where the side flaps 70 are folded inward such that the positions of the join portions 1ewl, 1ewr do not overlap with an overlap region W70 in the front-back direction (up-down direction) (see Fig. 10). In other words, the join portions 1ewl, 1ewr are positioned in the diapers 1 further to the lateral direction outside than the overlap region W70. In the overlap region W70, the thickness in the front-back direction is great due to the plural materials overlapped with each other in the front-back direction (up-down direction) . Thus were the join portions 1ewl, 1ewr to be formed in the overlap region W70, the thickness of the overlap region W70 would be even thicker due to the certain thickness of the join portions 1ewl, 1ewr themselves, making it difficult to fold the diapers 1 compactly. In contrast thereto, the diapers 1 can be folded thinly due to the join portions 1ewl, 1ewr not overlapping with the overlap region W70 in the diapers 1.

The diapers 1 having the side flaps 70 folded in by the fold-in means 130 are then compressed in the front-back direction (up-down direction) by the compressing means 140. The compressing means 140 has a function to compress the diapers 1 in the front-back direction (up-down direction) by nipping the entire diapers 1 from above and below, while conveying the diapers 1 in the MD. An ordinary belt conveyor may, for example, be employed as the compressing means 140. In the example of Fig. 8, an upper belt conveyor 140U and a lower belt conveyor 140D, arranged so as to face each other along the up-down direction, are provided as the compressing means 140. A predetermined size is maintained for the gap in the up-down direction between the upper belt conveyor 140U and the lower belt conveyor 140D, and the diapers 1 are compressed in the up-down direction by being nipped between the upper belt conveyor 140U and the lower belt conveyor 140D.

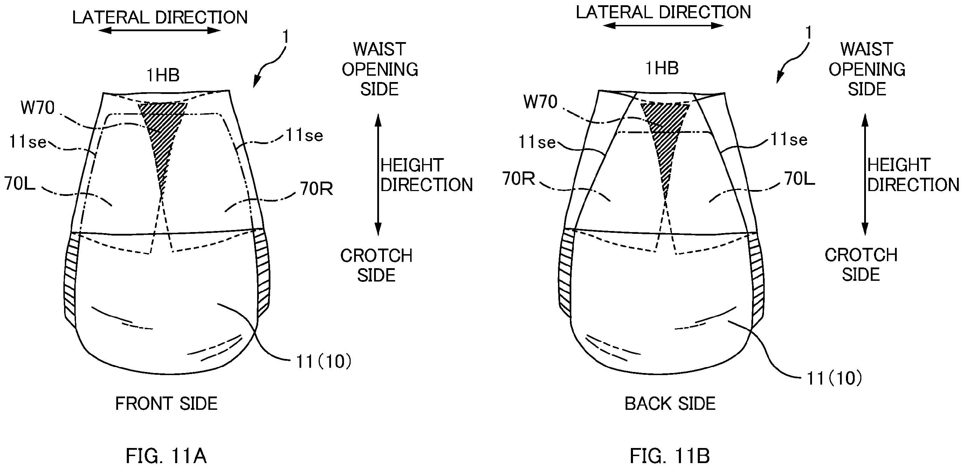

Fig. 11A is a schematic plan view of the diaper 1 in a state in which the side flaps 70 have been folded in, as viewed from the front side. Fig. 11B is a schematic plan view of the diaper 1 in a state in which the side flaps 70 have been folded in, as viewed from the back side. The region illustrated by shading in Fig. 11A and Fig. 11B is the overlap region W70 where the side flaps 70L, 70R that have been folded in are overlapped with each other in the front-back direction.

The front elastic region ERf is formed at the front side of the diapers 1 by the stretchable sheet 34 in the region at the height direction upper side of the absorbent main body 10, and contraction force in the lateral direction readily acts in this upper side region (see Fig. 2). This results in a shape in which, as illustrated in Fig. 11A, the absorbent main body 10 (the absorbent core 11) is contracted in the lateral direction at the height direction upper side. Then, due to the side flaps 70L, 70R being folded inward in the lateral direction along the profile of the two lateral direction side end portions 11se of the absorbent core 11, the side flaps 70L, 70R are folded deeply inward in the lateral direction at the height direction upper end side than at the height direction lower end side thereof. Namely, the surface area of portions on the waist opening side where the side flaps 70 are folded in between the absorbent main body in the up-down direction is greater than the surface area of portions on the crotch side where the side flaps 70 are folded in between the absorbent main body in the up-down direction. As a result, the overlap region W70 is formed at the height direction upper end side (waist opening side) of the side flaps 70L, 70R, as illustrated in Fig. 11A, and the overlap region W70 is not formed at the height direction lower end side (the crotch side).

When the side flaps 70L, 70R are not caused to overlap over the entire region in the height direction, and instead are only caused to overlap in a part region in the height direction, this suppresses an increase in thickness (width in the front-back direction) of the diapers 1 as a whole, thereby facilitating compact folding thereof. Moreover, due to compressing the diapers 1 using the compressing means 140 after the side flaps 70L, 70R have been folded in, the frictional force arising between the side flap 70L and the side flap 70R is greater where they are overlapped in the overlap region W70, rendering it more difficult to separate the side flaps 70L, 70R from each other. In particular, due to it being easier to maintain a folded-in state of the side flaps 70L, 70R at the waist opening side of the diapers 1, the waist opening 1HB is suppressed from inadvertently opening during packaging operations or the like.

Moreover, the back elastic region ERb is formed at the back side of the diapers 1 by the stretchable sheet 44 in the region at the height direction upper side of the absorbent main body 10, and contraction force in the lateral direction readily acts in this upper side region (see Fig. 2). Thus, similarly to at the front side, the side flaps 70L, 70R at the back side are also folded in deeply inward in the lateral direction more readily at the height direction upper side than at the lower side thereof. Moreover, due to a larger contraction force acting at the back elastic region ERb than at the front elastic region ERf, the absorbent main body 10 is contracted more in the lateral direction at the back side than at the front side. This means that, as illustrated in Fig. 11B, the side flaps 70L, 70R are folded in to positions more inward in the lateral direction at the back side of the diapers 1 than at the front side thereof. Thus when the diapers 1 are viewed with the side flaps 70 in the folded-in state, the profiles are clearly different from each other at the front side and the back side, enabling a user to easily discern the front and back (the front side and the back side) of the diapers 1. Note that the absorbent main body 10 may be configured so as to be contracted more in the lateral direction at the front side than at the back side, with the front and back of the diapers 1 also being easily discerned in such cases.

### ===Other Embodiments===

The embodiments described above are merely to facilitate understanding of the invention, and are not meant to be interpreted in a manner limiting the scope of the invention. The invention can of course be modified and improved without departing from the gist thereof and the invention includes functional equivalents of such modifications and improvements. For example, the following modifications may be made.

In the embodiment described above, an example is described in which a nonwoven fabric is employed as the material configuring the exterior back sheet 31 and the exterior back sheet 41. However, the material configuring each of these portions is not limited to such a nonwoven fabric. For example, a woven fabric may be employed therefor, or a sheet member that is not a woven fabric may be employed therefor.

In the embodiment described above, an example is described in which rubber threads are employed as the waist-gather elastic members 35 and the fit-gather elastic members 36. However, there is no limitation thereto. For example, band-shaped rubber may be employed therefor, or an elastic band-shaped nonwoven fabric or an elastic band-shaped resin film may be employed therefor.

### Reference Signs List

1: diaper (absorbent article, pull-on disposable diaper);
1ewr: join portion (right side); 1ewl: join portion (left side);
1HB: waist opening; 1HL: leg opening;
10: absorbent main body;
101e: end portions (height direction); lOse: end portion (lateral direction);
10fse: end portion (lateral direction/front side); 10bse: end portion (lateral direction/back side);
11: absorbent core; 111e: end portion (height direction); 11se:end portion (lateral direction)
13: front-face sheet; 15: back-face sheet; 15a: leak prevention sheet;
15b: exterior sheet;
20: leg-band member;
20le: end portion (height direction);
30: front exterior member;
30es: front side edge; 301: front band-shaped member;
31: exterior back sheet; 31f: folded-over section;
32: film sheet; 33: cover sheet; 34: stretchable sheet;
35: waist-gather elastic member; 36: fit-gather elastic member;
40: back exterior member;
40es: back side edge; 401: back band-shaped member;
41: exterior back sheet; 41f: folded-over section;
42: film sheet; 43: cover sheet; 44: stretchable sheet;
45: waist-gather elastic member; 46: fit-gather elastic member;
70: side flap;
70R: right side flap; 70Re: fold-in position;
70L: left side flap; 70Le: fold-in position;
70UR: upper region; 70MR: middle region; 70DR : lower region;
100: side flap fold-in mechanism;
110: conveying means;
120: pulling means;
120U: upward suction conveyor; 120D: downward suction conveyor;
130: fold-in means;
131: impeller; 131c: rotation shaft; 132: impeller drive section;
140: compressing means;
140U: upper belt conveyor; 140D: lower belt conveyor;
W70: overlap region;
CL10: predetermined position;
ERf: front elastic region; ERf: front elastic region;
LG leg gather; LSG: barrier cuff.

## Claims

1. A pull-on absorbent article (1) having a height direction, a lateral direction, and a front-back direction intersecting with each other, and comprising:
an absorbent main body (10) configured to absorb excrement;
a front exterior member (30) arranged at one end side of the absorbent main body; and
a back exterior member (40) arranged at another end side of the absorbent main body, with the front exterior member and the back exterior member joined together at a pair of join portions (lewr, 1ewl) , wherein:
at least a portion of the front exterior member (30) and the back exterior member (40) is folded in from outside to inside in the lateral direction;
each of the pair of join portions (lewr, 1ewl) where the front exterior member and the back exterior member are joined together is respectively positioned further outside in the lateral direction than an inside end (70Le, 70Re) in the lateral direction of the folded-in front exterior member (30) and the folded-in back exterior member (40); and
side flaps (70) are configured by the front exterior member (30) and the back exterior member (40), the side flaps being folded in at positions different to positions of the pair of join portions (lewr, 1ewl) .

2. The pull-on absorbent article of claim 1, wherein at least one of the front exterior member (30) and the back exterior member (40) includes a portion in the height direction where many layers of material are stacked and a portion in the height direction where few layers of material are stacked.

3. The pull-on absorbent article of claim 2, wherein at least one of the front exterior member (30) and the back exterior member (40) includes a portion at a middle region (70MR) between two end portions in the height direction where a number of stacked layers of the material is fewer than at the two height direction end portions.

4. The pull-on absorbent article of claim 3, wherein:
the absorbent main body (10) includes an absorbent core (11) configured by stacking layers of a material having liquid absorption properties; and
two end portions (11se) in the lateral direction of the absorbent core (11) include a portion that overlaps with the middle region (70MR) of the front exterior member (30) and the back exterior member (40).

5. The pull-on absorbent article of any one of claim 1 to claim 4, further comprising:
a pair of leg-band members (20) arranged at two sides of the absorbent main body (10) in the lateral direction, wherein
end portions (201e) in the height direction of the leg-band members (20) are positioned further inside in the height direction than end portions (101e) in the height direction of the absorbent main body (10) .

6. The pull-on absorbent article of any one of claim 1 to claim 5, wherein:
the absorbent main body (10) includes an absorbent core (11) configured from stacked layers of a material having liquid absorption properties;
at least one of the front exterior member (30) and the back exterior member (40) includes an elastic region that stretches and contracts along the lateral direction closer to a skin side than the absorbent core (11) .

7. The pull-on absorbent article of claim 6, wherein a sheet member capable of stretching and contracting in the lateral direction is provided in the elastic region.

8. The pull-on absorbent article of any one of claim 1 to claim 7, further comprising a portion where the front exterior member (30) and the back exterior member (40) folded in from outside to inside in the lateral direction at one side in the lateral direction, overlaps in the front-back direction with the front exterior member (30) and the back exterior member (40) folded in from the outside to the inside in the lateral direction at another side in the lateral direction.

9. The pull-on absorbent article of claim 8, wherein:
the pair of join portions (lewr, 1ewl) are positioned outside in the lateral direction of the portion where the front exterior member (30) and the back exterior member (40) folded in from the outside to the inside in the lateral direction at one side of the lateral direction overlaps in the front-back direction with the front exterior member (30) and the back exterior member (40) folded in from the outside to the inside in the lateral direction at the other side of the lateral direction.

10. The pull-on absorbent article of any one of claim 1 to claim 9, wherein:
a surface area of a portion where the front exterior member (30) and the back exterior member (40) are folded in between a front side and back side of the absorbent main body (10) in the front-back direction at a waist opening side in the height direction,
is larger than a surface area of a portion where the front exterior (30) member and the back exterior member (40) are folded in between a front side and back side of the absorbent main body (10) in the front-back direction at a crotch side in the height direction.

11. The pull-on absorbent article of any one of claim 1 to claim 10, wherein:
the absorbent main body (10) is contracted in the lateral direction by the elastic region that stretches and contracts along the lateral direction; and
an amount of contraction in the lateral direction of the absorbent main body at the back side is different to an amount of contraction in the lateral direction of the absorbent main body at the front side.

## Patentansprüche

1. Absorbierender Schlupfartikel (1), der eine Höhenrichtung, eine laterale Richtung und Vorn-hinten-Richtung aufweist, die einander schneiden, und der Folgendes umfasst:
einen absorbierenden Hauptkörper (10), der zur Absorption von Ausscheidungen konfiguriert ist;
ein vorderes Außenelement (30), das an einer Endseite des absorbierenden Hauptkörpers angeordnet ist; und
ein hinteres Außenelement (40), das an einer anderen Endseite des absorbierenden Hauptkörpers angeordnet ist, wobei das vordere Außenelement und das hintere Außenelement an einem Paar von Verbindungsteilen (lewr, lewl) miteinander verbunden sind, wobei:
mindestens ein Teil des vorderen Außenelements (30) und des hinteren Außenelements (40) von außen nach innen in der lateralen Richtung eingefaltet ist;
jedes des Paars von Verbindungsteilen (lewr, lewl), wo das vordere Außenelement und das hintere Außenelement miteinander verbunden sind, jeweils weiter außen in der lateralen Richtung positioniert ist als ein innenliegendes Ende (70Le, 70Re) in der lateralen Richtung des eingefalteten vorderen Außenelements (30) und des eingefalteten hinteren Außenelements (40); und
Seitenklappen (70) durch das vordere Außenelement (30) und das hintere Außenelement (40) konfiguriert sind, wobei die Seitenklappen an Positionen, die sich von den Positionen des Paars von Verbindungsteilen (lewr, lewl) unterscheiden, eingefaltet sind.

2. Absorbierender Schlupfartikel nach Anspruch 1, wobei mindestens eines von dem vorderen Außenelement (30) und dem hinteren Außenelement (40) ein Teil in der Höhenrichtung, wo viele Schichten an Material geschichtet sind, und ein Teil in der Höhenrichtung, wo wenige Schichten an Material geschichtet sind, einschließt.

3. Absorbierender Schlupfartikel nach Anspruch 2, wobei mindestens eines von dem vorderen Außenelement (30) und dem hinteren Außenelement (40) ein Teil an einem mittleren Bereich (70MR) zwischen zwei Endteilen in der Höhenrichtung einschließt, wo eine Anzahl von geschichteten Schichten des Materials kleiner ist als an den zwei Endteilen in der Höhenrichtung.

4. Absorbierender Schlupfartikel nach Anspruch 3, wobei:
der absorbierende Hauptkörper (10) einen Saugkern (11) einschließt, der durch Schichten von Schichten eines Materials, das Eigenschaften der Flüssigkeitsabsorption aufweist, konfiguriert ist; und
zwei Endteile (11se) in der lateralen Richtung des Saugkerns (11) ein Teil einschließen, das mit dem mittleren Bereich (70MR) des vorderen Außenelements (30) und des hinteren Außenelements (40) überlappt.

5. Absorbierender Schlupfartikel nach einem von Anspruch 1 bis Anspruch 4, der weiter Folgendes umfasst:
ein Paar von Beinabschlusselementen (20), die an zwei Seiten des absorbierenden Hauptkörpers (10) in der lateralen Richtung angeordnet sind, wobei
Endteile (201e) in der Höhenrichtung der Beinabschlusselemente (20) weiter innen in der Höhenrichtung positioniert sind als Endteile (101e) in der Höhenrichtung des absorbierenden Hauptkörpers (10).

6. Absorbierender Schlupfartikel nach einem von Anspruchs 1 bis Anspruch 5, wobei:
der absorbierende Hauptkörper (10) einen Saugkern (11) einschließt, der aus geschichteten Schichten eines Materials, das Eigenschaften der Flüssigkeitsabsorption aufweist, konfiguriert ist;
mindestens eines des vorderen Außenelements (30) und des hinteren Außenelements (40) einen elastischen Bereich einschließt, der sich entlang der lateralen Richtung, näher zu einer Hautseite als der Saugkern (11), dehnt und zusammenzieht.

7. Absorbierender Schlupfartikel nach Anspruch 6, wobei ein Lagenelement, das sich in der lateralen Richtung dehnen und zusammenziehen kann, in dem elastischen Bereich bereitgestellt ist.

8. Absorbierender Schlupfartikel nach einem von Anspruch 1 bis Anspruch 7, der weiter ein Teil umfasst, wo das vordere Außenelement (30) und das hintere Außenelement (40), die von außen nach innen in der lateralen Richtung an einer Seite in der lateralen Richtung eingefaltet sind, in der Vorn-hinten-Richtung mit dem vorderen Außenelement (30) und dem hinteren Außenelement (40), die von außen nach innen in der lateralen Richtung an einer anderen Seite in der lateralen Richtung eingefaltet sind, überlappen.

9. Absorbierender Schlupfartikel nach Anspruch 8, wobei:
das Paar von Verbindungsteilen (lewr, lewl) außen in der lateralen Richtung des Teils positioniert ist, wo das vordere Außenelement (30) und das hintere Außenelement (40), die von außen nach innen in der lateralen Richtung an einer Seite der lateralen Richtung eingefaltet sind, in der Vorn-hinten-Richtung mit dem vorderen Außenelement (30) und dem hinteren Außenelement (40), die von außen nach innen in der lateralen Richtung an der anderen Seite der lateralen Richtung eingefaltet sind, überlappen.

10. Absorbierender Schlupfartikel nach einem von Anspruch 1 bis Anspruch 9, wobei:
eine Oberflächenfläche eines Teils, wo das vordere Außenelement (30) und das hintere Außenelement (40) zwischen einer Vorderseite und Hinterseite des absorbierenden Hauptkörpers (10) in der Vorn-hinten-Richtung an einer Taillenöffnungsseite in der Höhenrichtung eingefaltet sind,
größer ist als eine Oberflächenfläche eines Teils, wo das vordere Außenelement (30) und das hintere Außenelement (40) zwischen einer Vorderseite und Hinterseite des absorbierenden Hauptkörpers (10) in der Vorn-hinten-Richtung an einer Schrittseite in der Höhenrichtung eingefaltet sind.

11. Absorbierender Schlupfartikel nach einem von Anspruch 1 bis Anspruch 10, wobei:
sich der absorbierende Hauptkörper (10) in der lateralen Richtung durch den elastischen Bereich zusammenzieht, der sich entlang der lateralen Richtung dehnt und zusammenzieht; und
sich ein Ausmaß des Zusammenziehens in der lateralen Richtung des absorbierenden Hauptkörpers an der Hinterseite von einem Ausmaß des Zusammenziehens in der lateralen Richtung des absorbierenden Hauptkörpers an der Vorderseite unterscheidet.

## Revendications

1. Article absorbant de type culotte (1) ayant une direction allant dans le sens de la hauteur, une direction allant dans le sens latéral, et une direction allant dans le sens avant-arrière se croisant les unes les autres, et comportant :
un corps principal absorbant (10) configuré pour absorber des excréments ;
un élément extérieur avant (30) agencé au niveau d'un côté d'extrémité du corps principal absorbant ; et
un élément extérieur arrière (40) agencé au niveau d'un autre côté d'extrémité du corps principal absorbant, l'élément extérieur avant et l'élément extérieur arrière étant assemblés ensemble au niveau d'une paire de parties d'assemblage (lewr, 1ewl), dans lequel :
au moins une partie de l'élément extérieur avant (30) et de l'élément extérieur arrière (40) est pliée vers l'intérieur depuis l'extérieur jusque dans l'intérieur dans la direction allant dans le sens latéral ;
chacune de la paire de parties d'assemblage (lewr, 1ewl) où l'élément extérieur avant et l'élément extérieur arrière sont assemblés ensemble est positionnée respectivement plus vers l'extérieur dans la direction allant dans le sens latéral par rapport à une extrémité côté intérieur (70Le, 70Re) dans la direction allant dans le sens latéral de l'élément extérieur avant plié vers l'intérieur (30) et l'élément extérieur arrière plié vers l'intérieur (40) ; et
des rabats latéraux (70) sont configurés par l'élément extérieur avant (30) et l'élément extérieur arrière (40), les rabats latéraux étant pliés vers l'intérieur au niveau de positions différentes des positions de la paire de parties d'assemblage (lewr, 1ewl).

2. Article absorbant de type culotte selon la revendication 1, dans lequel au moins l'un parmi l'élément extérieur avant (30) et l'élément extérieur arrière (40) comprend une partie dans la direction allant dans le sens de la hauteur où de nombreuses couches de matériau sont empilées et une partie dans la direction allant dans le sens de la hauteur où quelques couches de matériau sont empilées.

3. Article absorbant de type culotte selon la revendication 2, dans lequel au moins l'un parmi l'élément extérieur avant (30) et l'élément extérieur arrière (40) comprend une partie au niveau d'une région intermédiaire (70MR) entre deux parties d'extrémité dans la direction allant dans le sens de la hauteur où un certain nombre de couches de matériau empilées est inférieur par rapport au niveau des deux parties d'extrémité dans la direction allant dans le sens de la hauteur.

4. Article absorbant de type culotte selon la revendication 3, dans lequel :
le corps principal absorbant (10) comprend une partie centrale absorbante (11) configurée en empilant des couches d'un matériau ayant des propriétés d'absorption de liquide ; et
deux parties d'extrémité (11se) dans la direction allant dans le sens latéral de la partie centrale absorbante (11) comprennent une partie qui chevauche la région intermédiaire (70MR) de l'élément extérieur avant (30) et de l'élément extérieur arrière (40).

5. Article absorbant de type culotte selon l'une quelconque des revendications allant de la revendication 1 à la revendication 4, comportant par ailleurs :
une paire d'éléments formant bande au niveau de jambes (20) agencés au niveau de deux côtés du corps principal absorbant (10) dans la direction allant dans le sens latéral, dans lequel
des parties d'extrémité (201e) dans la direction allant dans le sens de la hauteur des éléments formant bande au niveau de jambes (20) sont positionnées plus encore vers l'intérieur dans la direction allant dans le sens de la hauteur par rapport à des parties d'extrémité (101e) dans la direction allant dans le sens de la hauteur du corps principal absorbant (10).

6. Article absorbant de type culotte selon l'une quelconque des revendications allant de la revendication 1 à la revendication 5, dans lequel :
le corps principal absorbant (10) comprend une partie centrale absorbante (11) configurée à partir de couches empilées d'un matériau ayant des propriétés d'absorption de liquide ;
au moins l'un parmi l'élément extérieur avant (30) et l'élément extérieur arrière (40) comprend une région élastique qui s'étire et se contracte le long de la direction allant dans le sens latéral plus proche d'un côté peau par rapport à la partie centrale absorbante (11).

7. Article absorbant de type culotte selon la revendication 6, dans lequel un élément formant feuille en mesure de s'étirer et de se contracter dans la direction allant dans le sens latéral est mis en œuvre dans la région élastique.

8. Article absorbant de type culotte selon l'une quelconque des revendications allant de la revendication 1 à la revendication 7, comportant par ailleurs une partie où l'élément extérieur avant (30) et l'élément extérieur arrière (40) pliés vers l'intérieur depuis l'extérieur jusque dans l'intérieur dans la direction allant dans le sens latéral au niveau d'un côté dans la direction allant dans le sens latéral, chevauchent dans la direction allant dans le sens avant-arrière l'élément extérieur avant (30) et l'élément extérieur arrière (40) pliés vers l'intérieur depuis l'extérieur jusque dans l'intérieur dans la direction allant dans le sens latéral au niveau d'un autre côté dans la direction allant dans le sens latéral.

9. Article absorbant de type culotte selon la revendication 8, dans lequel :
les parties d'assemblage de la paire de parties d'assemblage (lewr, 1ewl) sont positionnées à l'extérieur dans la direction allant dans le sens latéral de la partie où l'élément extérieur avant (30) et l'élément extérieur arrière (40) pliés vers l'intérieur depuis l'extérieur jusque dans l'intérieur dans la direction allant dans le sens latéral au niveau d'un côté de la direction allant dans le sens latéral chevauchent dans la direction allant dans le sens avant-arrière l'élément extérieur avant (30) et l'élément extérieur arrière (40) pliés vers l'intérieur depuis l'extérieur jusque dans l'intérieur dans la direction allant dans le sens latéral au niveau de l'autre côté de la direction allant dans le sens latéral.

10. Article absorbant de type culotte selon l'une quelconque des revendications allant de la revendication 1 à la revendication 9, dans lequel :
une surface d'une partie où l'élément extérieur avant (30) et l'élément extérieur arrière (40) sont pliés entre un côté avant et un côté arrière du corps principal absorbant (10) dans la direction allant dans le sens avant-arrière au niveau d'un côté d'ouverture au niveau de la taille dans la direction allant dans le sens de la hauteur,
est supérieure par rapport à une surface d'une partie où l'élément extérieur avant (30) et l'élément extérieur arrière (40) sont pliés entre un côté avant et un côté arrière du corps principal absorbant (10) dans la direction allant dans le sens avant-arrière au niveau d'un côté au niveau de l'entrejambe dans la direction allant dans le sens de la hauteur.

11. Article absorbant de type culotte selon l'une quelconque des revendications allant de la revendication 1 à la revendication 10, dans lequel :
le corps principal absorbant (10) est contracté dans la direction allant dans le sens latéral par la région élastique qui s'étire et se contracte le long de la direction allant dans le sens latéral ; et
une quantité de contraction dans la direction allant dans le sens latéral du corps principal absorbant au niveau du côté arrière est différente d'une quantité de contraction dans la direction allant dans le sens latéral du corps principal absorbant au niveau du côté avant.
